# EUROPEAN PATENT SPECIFICATION

(11) **EP 4 171 577 B1**
(45) Date of publication and mention of the grant of the patent: **18.02.2026**
(21) Application number: 21828315.8
(22) Date of filing: 22.06.2021
(51) Int. Cl.: A61K 31/713, A61K 47/55, A61K 9/127, C07C 403/24

(54) **PROCESS FOR SYNTHESIZING TARGETING MOLECULES**
VERFAHREN ZUR SYNTHESE VON ZIELMOLEKÜLEN
PROCÉDÉ DE SYNTHÈSE DE MOLÉCULES DE CIBLAGE

(30) Priority: 24.06.2020 US 202063043516 P
(43) Date of publication of application: 03.05.2023
(73) Proprietor: Bristol-Myers Squibb Company, Princeton, NJ 08543 (US)
(72) Inventor: SHI, Zhongping, Princeton, New Jersey 08543 (US); TAN, Yichen, Princeton, New Jersey 08543 (US); LORA GONZALEZ, Federico, Princeton, New Jersey 08543 (US); CARRASQUILLO-FLORES, Ronald, Princeton, New Jersey 08543 (US); SMITH, Michael, J., Princeton, New Jersey 08543 (US)
(74) Representative: Mewburn Ellis LLP
(86) International application number: PCT/US2021/038522
(87) International publication number: WO 2021/262743

(56) References cited:
- WO-A2-2012/170952
- US-A1- 2013 017 249
- US-A1- 2013 171 240
- US-A1- 2018 297 938
- US-A1- 2019 275 158
- US-B2- 8 664 376

## Description

### FIELD OF THE INVENTION

The present application provides processes for synthesizing targeting molecules comprising a retinoid moiety useful in the synthesis of fat-soluble compounds for targeting and enhancing activity of therapeutic molecules, including siRNA.

### BACKGROUND OF THE INVENTION

It is known in the art that creating molecules capable of targeting a specific receptor, tissue type, or organ of interest is of great advantage. When specificity is low there is limited efficacy, higher amounts of administered therapeutics needed, and an increase in off-target effects. Molecular scaffolds capable of targeting the delivery of a therapeutic agent are one way to overcome the problem. One such scaffold, comprising the basic structure (targeting moiety)ⱼ-linker-(targeting moiety)ₖ, wherein the targeting moiety is a retinoid radical j and k are independently 0, 1, 2, or 3; and the linker is PEG-like, is disclosed in U.S. Patent No. 9,393,315. WO 2012/170952 A2 and US 2013/0171240 A1 describe molecules comprising a retinoid moiety and methods of their synthesis.

This scaffold is used to facilitate drug delivery to a target cell having a specific receptor or activation/binding for a retinoid. One example of a molecule using this scaffold is N¹,N¹⁹-bis((S,23E,25E,27E,29E)-16-((2E,4E,6E,8E)-3,7-dimethyl-9-(2,6,6-trimethylcyclohex-1-en-1-yl)nona-2,4,6,8-tetraenamido)-24,28-dimethyl-15,22-dioxo-30-(2,6,6-trimethylcyclohex-1-en-1-yl)-4,7,10-trioxa-14,21-diazatriaconta-23,25,27,29-tetraen-1-yl)-4,7,10,13,16-pentaoxanonadecanediamide ("DiVa"). Previous synthetic strategies for producing DiVa and similar compounds were plagued with low yield, a high number of side products, and lack of availability of high quality starting materials.

There remains a need for a synthetic process to produce these retinoid containing targeting molecules that facilitates increased product yield, low by-product yield, and uses more accessible starting materials.

### SUMMARY OF THE INVENTION

In one aspect, the present application provides a process for producing DiVa.

In one embodiment the present application provides a process for synthesizing a compound of Formula I 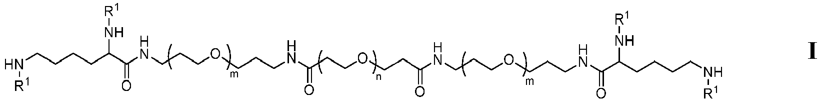 wherein, R¹ is a retinoid radical;
m is an integer from 1-6; and
n is an integer from 1-10
the process comprising,
   a) reacting a compound of Formula II 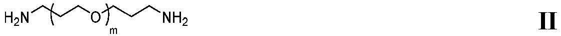 wherein, m is an integer 1-6;
      with a compound of Formula III 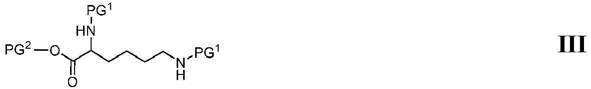
      wherein, PG¹ and PG² are each independently a protecting group;
      to form a compound of Formula IV 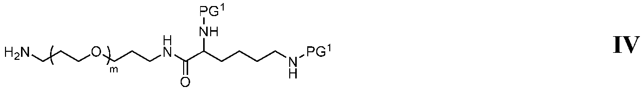
      wherein m is an integer 1-6; and
      PG¹ is a protecting group
   b) reacting a compound of Formula IV with a compound of Formula V 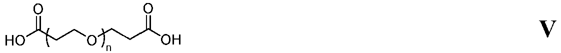 wherein, n is an integer 1-10;
      to form a compound of Formula VI 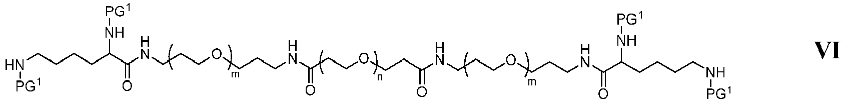
      wherein, m is an integer 1-6;
      n is an integer 1-10; and
      PG¹ is a protecting group;
   c) reacting a compound of Formula VI under hydrogenation conditions to form a compound of Formula VII 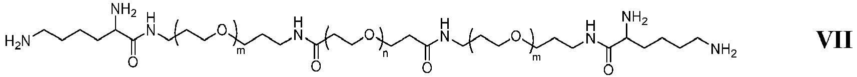 wherein m is an integer 1-6; and
      n is an integer 1-10; and
   d) reacting the compound of Formula VII under coupling conditions with a retinoid to form a compound of Formula I.

In a further embodiment the retinoid is selected from vitamin A, retinoic acid, tretinoin, adapalene, 4-hydroxy(phenyl)retinamide, retinyl palmitate, retinal, saturated retinoic acid, all-trans retinoic acid, and saturated demethylated retinoic acid.

In yet another further embodiment the retinoid is retinoic acid.

In a further embodiment the hydrogenation conditions of step c) comprise reacting a compound of Formula I with H₂ and Pd/C.

In another further embodiment m is 3.

In yet another further embodiment n is 5.

In another embodiment each PG¹ and PG² is independently selected from the group consisting of carboxybenzyl, *p*-methoxybenzyl carbonyl, t-butyloxycarbonyl, 9-fluorenylmethyloxycarbonyl, acetyl, trifluoroacetyl, benzoyl, benzyl, carbamate, p-methoxybenzyl, 3,4-dimethoxybenzyl, p-methoxybenzyl, tosyl, trichloroethyl chloroformate, (4-nitrophenyl)sulfonyl, methyl, ethyl, propyl, n-butyl, t-butyl, succinimide, 2,6-dimethylphenol, 2,6-diisopropylphenol, 2,6-di-tert-butylphenol, trimethylsilyl, allyl, 1,1-dimethylallyl, 2,2,2-trifluoro-ethyl, phenyl, and 4-methoxybenzyl.

In a further embodiment each PG¹ is carboxybenzyl.

In another embodiment PG² is succinimide.

In a further embodiment the compound of Formula II is

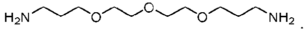

In another embodiment the compound of Formula III is

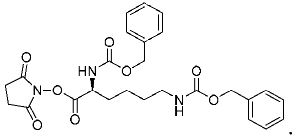

In a further embodiment step a) is done at between -20 °C and 0 °C.

In a further embodiment the yield of a) is at least about 60%. In another embodiment the yield of step b) is at least about 70%.

In a further embodiment in step a) the compound of Formula II is used in between about 5 and about 20 equivalents and the compound of Formula III is used in about 1 equivalent.

In another embodiments step a) further comprises the addition of citric acid after formation of the compound of Formula IV.

In a further embodiment the compound of Formula I is

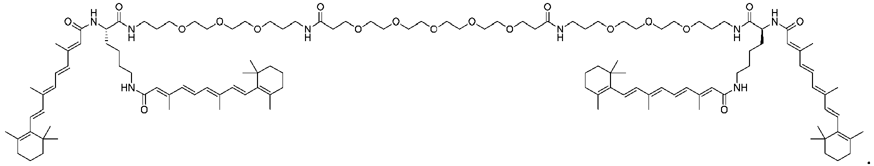

In a further embodiment the compound of Formula I is produced in at least about 50% yield from the compound of Formula III.

In a further embodiment the compound of Formula IV is produced in a ratio of greater than about 9:1 with a compound of Formula VIII

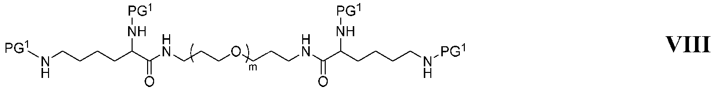

In a further embodiment the compound of Formula IV is produced in a ratio of greater than 12:1 with a compound of Formula VIII.

A compound of Formula I can be prepared by the process of steps a) -d).

### DETAILED DESCRIPTION OF THE INVENTION

Throughout the specification and the appended claims, a given chemical formula or name shall encompass all stereo and optical isomers and racemates thereof where such isomers exist. All chiral (enantiomeric and diastereomeric) and racemic forms are contemplated herein. Many geometric isomers of C=C double bonds, C=N double bonds, ring systems, and the like can also be present in the compounds. Cis- and trans- (or E- and Z-) geometric isomers of the compounds are described and may be isolated as a mixture of isomers or as separated isomeric forms. The present compounds can be isolated in optically active or racemic forms. Optically active forms may be prepared by resolution of racemic forms or by synthesis from optically active starting materials. When enantiomeric or diastereomeric products are prepared, they may be separated by conventional methods, for example, by chromatography or fractional crystallization.

Depending on the process conditions the end products of the present invention are obtained either in free (neutral) or salt form. Both the free form and the salts of these end products are contemplated herein. If so desired, one form of a compound may be converted into another form. A free base or acid may be converted into a salt; a salt may be converted into the free compound or another salt; a mixture of isomeric compounds may be separated into the individual isomers. Compounds herein, the free form and salts thereof, may exist in multiple tautomeric forms, in which hydrogen atoms are transposed to other parts of the molecules and the chemical bonds between the atoms of the molecules are consequently rearranged. It should be understood that all tautomeric forms are also contemplated.

The term "stereoisomer" refers to isomers of identical constitution that differ in the arrangement of their atoms in space. Enantiomers and diastereomers are examples of stereoisomers. The term "enantiomer" refers to one of a pair of molecular species that are mirror images of each other and are not superimposable. The term "diastereomer" refers to stereoisomers that are not mirror images. The term "racemate" or "racemic mixture" refers to a composition composed of equimolar quantities of two enantiomeric species, wherein the composition is devoid of optical activity. It is well understood in the art that controlling the stereochemistry of a product is possible by choosing the stereochemistry of the starting materials, and that the stereochemistry of a product can be changed by changing the stereochemistry of the starting material. It is also well understood in the art how to separate a racemic mixture such that the stereochemical purity of a product is > 99%.

The symbols "R" and "S" represent the configuration of substituents around a chiral carbon atom(s). The isomeric descriptors "R" and "S" are used as described herein for indicating atom configuration(s) relative to a core molecule and are intended to be used as defined in the literature (IUPAC Recommendations 1996, Pure and Applied Chemistry, 68, 2193-2222 (1996)).

The term "chiral" refers to the structural characteristic of a molecule that makes it impossible to superimpose it on its mirror image. The term "homochiral" refers to a state of enantiomeric purity. The term "optical activity" refers to the degree to which a homochiral molecule or nonracemic mixture of chiral molecules rotates a plane of polarized light.

Abbreviations as used herein, are defined as follows: "°C" for degrees Celsius, "eq" for equivalent or equivalents, "g" for gram or grams, "mg" for milligram or milligrams, "L" for liter or liters, "mL" for milliliter or milliliters, "µL" for microliter or microliters, "N" for normal, "M" for molar, "mmol" for millimole or millimoles, "min" for minute or minutes, "h" for hour or hours, "rt" for room temperature, "RT" for retention time, "conc." for concentrate, "sat" or "saturated " for saturated, "MW" for molecular weight, "ee" for enantiomeric excess, "MS" or "Mass Spec" for mass spectrometry, "ESI" for electrospray ionization mass spectroscopy, "HR" for high resolution, "HRMS" for high resolution mass spectrometry, "LCMS" for liquid chromatography mass spectrometry, "HPLC" for high pressure liquid chromatography, "NMR" for nuclear magnetic resonance spectroscopy, "¹H" for proton, and "D", "L" "α", "β", "R", "S", "E", and "Z" are stereochemical designations familiar to one skilled in the art.

Provided herein is a process for the synthesis of a targeting molecule comprising the basic structure (R¹)ⱼ-linker-(R¹)ₖ, wherein R¹ is a retinoid; j and k are independently 0, 1, 2, or 3; and the linker is PEG-like. In an embodiment the process is a process for synthesizing a compound of Formula I. In an embodiment the process is a process of synthesizing a compound of Formula I starting from a compound of Formula III.

### Step a)

In an embodiment step a) comprises
reacting a compound of Formula II 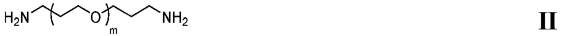
wherein, m is an integer 1-10;
with a compound of Formula III 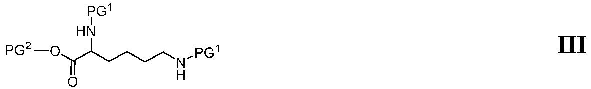
wherein, PG² is a protecting group;
to form a compound of Formula IV

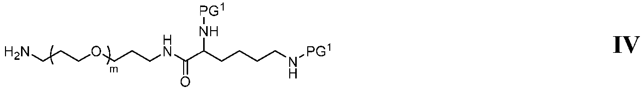

In an embodiment m is an integer 1-10. In another embodiment m is an integer 1-9, 1-8, 1-7, 1-6, 1-5, 1-4, 1-3, or 1-2. In an embodiment m is an integer 1-6. In another embodiment m is an integer 2-10, 3-10, 4-10, 5-10, 6-10, 7-10, 8-10, or 9-10. In another embodiment m is an integer 2-9, 3-8, 4-7, or 5-6. In an embodiment m is 1. In an embodiment m is 2. In an embodiment m is 3. In an embodiment m is 4. In an embodiment m is 5. In an embodiment m is 6. In an embodiment m is 7. In an embodiment m is 8. In an embodiment m is 9. In an embodiment m is 10.

In an embodiment PG¹ is an amine protecting group. In another embodiment PG¹ is selected from the group consisting of carboxybenzyl (CBz), p-methoxybenzyl carbonyl (Moz), t-butyloxycarbonyl (BOC), 9-fluorenylmetholoxycarbonyl (Fmoc), Acetyl (Ac), trifluoroacetyl, benzoyl (Bz), benzyl (Bn), carbamate, p-methoxybenzyl (PMB), 3,4-dimethoxybenzyl, p-methoxybenzyl, tosyl (Ts), tricloroethyl chloroformate (Troc), (4-nitrophenyl)sulfonyl (Nosyl). In an embodiment PG¹ is carboxybenzyl.

In an embodiment PG² is a carboxylic acid protecting group. In an embodiment PG² is selected from the group consisting of methyl, ethyl, propyl, n-butyl, t-butyl, Bn, succinimide (Su), 2,6-dimethylphenol, 2,6-diisopropylphenol, 2,6-di-tert-butylphenol, trimethylsilyl, allyl, 1,1-dimethylallyl, 2,2,2-trifluoro ethyl, phenyl (Ph), and 4-methoxybenzyl, In an embodiment PG² is succinimide.

In an embodiment PG¹ is carboxybenzyl and PG² is succinimide. In an embodiment PG¹ is carboxybenzyl, PG² is succinimide, m is 3.

In an embodiment step a) takes place in a solvent. In an embodiment the solvent is a combination of solvents. In an embodiment at least one solvent is polar aprotic. In another embodiment more than one of the solvents is polar aprotic. In an embodiment the solvent(s) are selected from dichloromethane (DCM), ethyl acetate (EtOAc), tetrahydrofuran (THF), acetone, *N*,*N*-dimethylformamide (DMF), acetonitrile, and dimethyl sulfoxide (DMSO). In an embodiment the solvent is a combination of DCM and EtOAc.

In an embodiment step a) takes place between about -30 °C and about 0 °C. In an embodiment the process takes places between about -25 °C and about 0 °C, between about -20 °C and about 0 °C, between about -15 °C and about 0 °C, or between about -10 °C and about 0 °C. In an embodiment step a) takes place between about -30 °C and about -5 °C, between about - 30 °C and about -15 °C, or between about -30 °C and about -25 °C. In an embodiment step a) takes place between about -25° C and about -5 °C, between about -20 °C and -10 °C, or between -18 °C and -12 °C. In an embodiment step a) takes place between about -20 °C and about -12 °C. In an embodiment step a) takes place at about -20 °C, about -15 °C, about -10 °C, about -5 °C, or about 0 °C. In an embodiment step a) takes place at less than about 0 °C. In an embodiment step a) takes place at less than about -5 °C. In an embodiment step a) takes place at less than about -10 °C. In an embodiment step a) takes place at less than about -15 °C.

In an embodiment the compound of Formula IV is produced in between about 70 % and 95% yield from step a). In an embodiment the compound of Formula IV is produced, in between about 70% and 85%, or in between about 70% and 75% yield. In another embodiment the compound of Formula IV is produced in between about 80% and about 95%, or in between about 90% and 95% yield. In another embodiment the compound of Formula IV is produced in about 70%, about 75%, about 80%, about 85%, about 90%, or about 95% yield. In a further embodiment the compound of Formula IV is produced in about 80% yield. In an embodiment the compound of Formula IV is produced in at least about 65% yield, in at least 70% yield, in at least 75% yield, in at least about 80% yield, or at least about 85% yield from step a). In another embodiment Formula IV is produced in at least about 80% yield from step a).

In an embodiment step a) comprises reacting about 10 eq. of a compound of Formula II with about 1 eq. of a compound of Formula III. In an embodiment step a) comprises reacting about 9 eq. of a compound of Formula II with about 1 eq. of a compound of Formula III, about 8 eq. of a compound of Formula II with about 1 eq. of a compound of Formula III, about 7 eq. of a compound of Formula II with about 1 eq. of a compound of Formula III, about 6 eq. of a compound of Formula II with about 1 eq. of a compound of Formula II, or about 5 eq. of a compound of Formula II with about 1 eq. of a compound of Formula III.

In an embodiment step a) comprises reacting less than about 8 eq. of a compound of Formula II with about 1 eq. of a compound of Formula III. In an embodiment step a) comprises reacting less than about 7 eq. of a compound of Formula II with about 1 eq. of a compound of Formula III. In an embodiment step a) comprises reacting less than about 6 eq. of a compound of Formula II with about 1 eq. of a compound of Formula III.

In a further embodiment the compound of Formula IV is produced in a ratio of greater than 9:1 with a compound of Formula VIII. In an embodiment the compound of Formula IV is produced in a ratio of greater than about greater than about 8:1, greater than about 9:1, greater than about 10:1, greater than about 11:1, greater than about 12:1, greater than about 13:1, greater than about 14:1, or greater than about 15:1 with a compound of Formula VIII. In a further embodiment the compound of Formula IV is produced in a ratio of about 9:1 with a compound of Formula VIII. In an embodiment the compound of Formula IV is produced in a ratio of about 8:1, about 9:1, about 10:1, about 11:1, about 12:1, about 13:1, about 14:1, or about 15:1 with a compound of Formula VIII.

In an embodiment step a) is a selective mono-amidation of diamine CAS # 4246-51-9 with double-Z-lysine derivative CAS # 21160-83-8. In an embodiment reacting a compound of Formula III with a compound of Formula II in step a) comprises slow addition of a solution of Formula III to a solution of Formula II. In an embodiment reacting a compound of Formula III with a compound of Formula II in step a) comprises sub-surface addition of a solution of Formula III to a solution of Formula II.

### Step b

In an embodiment step b comprises reacting a compound of Formula IV with a compound of Formula V 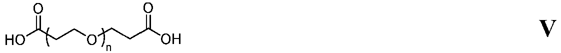 wherein, n is an integer 1-10;
to form a compound of Formula VI 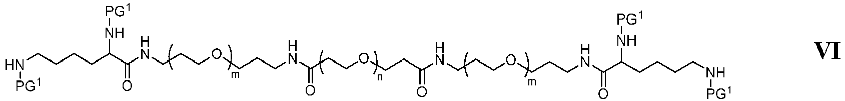
wherein, m is 1-6;
n is 1-10; and
PG¹ is a protecting group.

In an embodiment m, n, PG¹, and PG² are as described for step a). In an embodiment, PG¹ is carboxybenzyl, PG² is succinimide, m is 3, and n is 5.

In an embodiment step b) takes place in a solvent. In an embodiment at least one solvent is polar aprotic. In an embodiment the solvent(s) is selected from dichloromethane (DCM), ethyl acetate (EtOAc), tetrahydrofuran (THF), acetone, *N*,*N*-dimethylformamide (DMF), acetonitrile, and dimethyl sulfoxide (DMSO). In an embodiment step b) takes place in DCM.

In an embodiment step b) takes place between about 0 °C and about 30 °C. In an embodiment the process takes places between about 10 °C and about 30 °C, or between about 20 °C and about 30 °C. In an embodiment step b) takes place between about 0 °C and about 25 °C, , between about 0 °C and about 15 °C, or between about 0 °C and about 5 °C. In an embodiment step b) takes place between about 15 °C and about 20 °C. In an embodiment step b) takes place at about 30 °C, about 25 °C, about 20 °C, about 15 °C, about 10 °C, about 5 °C, or about 0 °C. In an embodiment step b) takes place at less than about 30 °C. In an embodiment step b) takes place at less than about 25 °C. In an embodiment step b) takes place at about 20 °C. In an embodiment step b) takes place at about room temperature.

In an embodiment the compound of Formula VI is produced in between about 70 % and 99% yield from step b). In an embodiment the compound of Formula VI is produced in between about 70% and 95%, in between about 70% and 90%, in between about 70% and 85%, in between about 70% and 80%, or in between about 70% and 75% yield. In another embodiment the compound of Formula VI is produced in between about 75% and about 99%, in between about 80% and about 99%, in between about 85% and about 99%, in between about 90% and 99% yield, or in between about 95% and about 99% yield from step b). In another embodiment the compound of Formula VI is produced in about 70%, about 75%, about 80%, about 85%, about 90%, about 95%, or about 99% yield from step b). In a further embodiment the compound of Formula VI is produced in about 90% yield. In a further embodiment the compound of Formula VI is produced in about 95% yield.

In an embodiment the compound of Formula VI is produced in at least about 65% yield, in at least about 70% yield, in at least about 75% yield, in at least about 80% yield, or at least about 85% yield from step b). In another embodiment Formula VI is produced in at least about 80% yield from step b).

In an embodiment step b) is an 3-(((ethylimino)methylene)amino)-N,N-dimethylpropan-1-amine (EDAC) mediated double amidation of diacid CAS: 439114-13-3.

### Step c

c) reacting a compound of Formula VI under hydrogenation conditions to form a compound of Formula VII 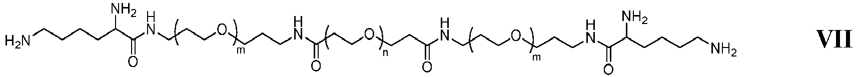 wherein m is an integer 1-10; and
n is an integer1-10.

In an embodiment m and n are as described in step a). In an embodiment m is 3 and n is 5.

In an embodiment step c) takes place in a solvent. In an embodiment the solvent is a combination of solvents. In another embodiment at least one solvent is polar protic. In another embodiment at least one solvent is polar aprotic. In another embodiment the solvent(s) is selected from the group consisting of methanol, ethanol, ethyl acetate, cyclohexane, methylcyclohexane, benzene, petroleum ether, ligroin, 2-methyltetrahydrofuam, acetone, tetrahydrofuran, dimethyl acetamide, N-methy-pyrrolidine and DMF.

In an embodiment the hydrogenation conditions comprise a hydrogenation catalyst. In an embodiment the hydrogenation catalyst is selected from palladium, rhenium, rhodium, ruthenium, platinum, or raney nickel. Other hydrogenation catalysts typical in the art that could be applied are outlined in Wang, D., et al. Chem. Rev. 2015, 115, 13, 6621-6686.

In an embodiment the hydrogenation catalyst is on a support. In an embodiment the support is selected from carbon, alumina, alkaline earth carbonates, clay, ceramic, pumice, or celite. In an embodiment the support is carbon.

In an embodiment the hydrogenation catalyst is palladium and the support is carbon (palladium on carbon or Pd/C).

In an embodiment the hydrogenation conditions comprise a hydrogen donor. In an embodiment the hydrogen donor is selected from 1-methyl-1,4-cyclohexadiene and 1,4-cyclohexadine. Other hydrogen donors that can be applied are outlined in Wang, D. et al. Chem. Rev. 2015, 115, 13, 6621-6686.

It was discovered that dissolved oxygen stalls the hydrogenation reaction due to side reaction with hydrogen donor. It was also discovered that CO₂ generation during the hydrogenation reaction stalls reaction due to catalyst poisoning. Thus, in an embodiment step c) is performed in a reaction vessel with an inert environment. In another embodiment the reaction vessel is sparged with N₂ before the reaction. In another embodiment the reaction vessel is sparged throughout the hydrogenation reaction with N₂.

In an embodiment step c) takes place between about 30 °C and about 60 °C. In an embodiment the process takes places between about 40 °C and about 60 °C, or between about 50 °C and about 60 °C. In an embodiment step c) takes place between about 30 °C and about 50 °C, or between about 30 °C and about 40 °C. In an embodiment step c) takes place between about 25° C and about 55 °C, between about 30 °C and about 50 °C, between about 35 °C and about 45 °C, or between about 38 °C and about 42 °C. In an embodiment step c) takes place between about 40 °C and about 45 °C. In an embodiment step c) takes place at about 30 °C, about 35 °C, about 40 °C, about 45 °C, about 50 °C, about 55 °C, or about 60 °C. In an embodiment step c) takes place at less than about 60 °C. In an embodiment step c) takes place at less than about 50 °C. In an embodiment step c) takes place at about 45 °C.

In an embodiment the compound of Formula VII is produced in between about 70 % and 99% yield from step b). In an embodiment the compound of Formula VII is produced in between about 70% and 99%, in between about 70% and 95%, in between about 70% and 90%, in between about 70% and 85%, in between about 70% and 80%, or in between about 70% and 75% yield. In another embodiment the compound of Formula IV is produced in between about 75% and about 99%, in between about 80% and about 99%, in between about 85% and about 99%, in between about 90% and 99% yield, or in between about 95% and about 99% yield from step b).

In another embodiment the compound of Formula VII is produced in about 70%, about 75%, about 80%, about 85%, about 90%, about 95%, about 96%, about 97%, about 98%, or about 99% yield from step b). In a further embodiment the compound of Formula VII is produced in about 90% yield. In a further embodiment the compound of Formula VII is produced in about 95% yield. In another embodiment the compound of Formula VII is produced in about 98% yield. In another embodiment the compound of Formula VII is produced in about 99% yield.

In an embodiment the compound of Formula VII is produced in at least about 65% yield, in at least about 70% yield, in at least about 75% yield, in at least about 80% yield, at least about 85%, at least about 90% yield, at least about 95% yield, at least about 98% yield, or at least about 99% yield from step b). In another embodiment Formula VII is produced in at least about 80% yield from step b).

In an embodiment step c) is a hydrogenation reaction. In an embodiment step c) is a heterogeneous palladium-catalyzed transfer hydrogenation of a compound of Formula VI.

### Step d

In an embodiment step d comprises reacting the compound of Formula VII with R¹ to form a compound of Formula I.

In an embodiment m and n are as described in step a). In an embodiment m is 3 and n is 5.

In a further embodiment R¹ is selected from vitamin A, retinoic acid, tretinoin, adapalene, 4-hydroxy(phenyl)retinamide, retinyl palmitate, retinal, all-trans retinoic acid, saturated retinoic acid, and saturated demethylated retinoic acid. In an embodiment the retinoid is a retinoic acid. In an embodiment the retinoid is all-trans retinoic acid.

In an embodiment the coupling conditions comprise an activation agent. In an embodiment the activation agent is selected from the group comprising 2-(1H-Benzotriazole-1-yl)-1,1,3,3-tetramethylaminium tetrafluoroborate (TBTU), (2-(1H-benzotriazol-1-yl)-1,1,3,3-tetramethyluronium hexafluorophosphate (HBTU), O-(7-Azabenzotriazole-1-yl)-N,N,N',N'-tetramethyluronium tetrafluoroborate (TATU), and 4-(4,6-dimethoxy-1,3,5-triazin-2-yl)-4-methyl-morpholinium chloride (DMTMM).

In an embodiment the coupling conditions comprise a base. In an embodiment the base is any base with a pKb of ≥8. In an embodiment the base is selected from trimethylamine, sodium hydroxide, *N*-methylmorpholine (NMM), 1-methylimidazole (NMI) and *N,N-*diisopropylethylamine (DiPEA), and potassium hydroxide.

In an embodiment step d) further comprises purification of the compound of Formula I by chromatography. In an embodiment the chromatography is done on a silica column. In a further embodiment the silica column is a C¹⁸ column. In an embodiment the silica column is treated with a solvent gradient. In an embodiment the solvent gradient comprises 20-40% ethanol in methanol.

It was discovered that compounds of Formula I degrade at light 500 nm or below. Thus, in an embodiment the compound of Formula I is not accessible by light 500 nm or lower in step d). In an embodiment the coupling conditions comprise excluding light 500 nm or lower. In an embodiment the purification of the compound of Formula I comprises excluding light 500 nm or lower.

It was discovered that compounds of Formula I oxidize when exposed to the atmosphere. Thus, in an embodiment the compound of Formula I is not accessible oxygen in step d). In an embodiment step d) is performed in a reaction vessel with an inert environment. In another embodiment the reaction vessel is sparged with N₂ before the reaction. In another embodiment step d) further comprises charging the reaction vessel with butylated hydroxytoluene (BHT). In another embodiment step d) further comprises a solvent swap into ethanol, and concentration to afford a final 35 wt% solution of a compound of Formula I with 400 -1500 ppm BHT to scavenge oxygen.

It was discovered that compounds of Formula I are relatively unstable above 45 °C. Thus, in an embodiment step d) takes place between about 25 °C and about 45 °C. In an embodiment the process takes places between about 35 °C and about 45 °C or between about 40 and 45 °C. In an embodiment step d) takes place between about 25 °C and about 40 °C, between about 25 °C and about 35 °C, or between about 25 °C and about 30 °C. In an embodiment step d) takes place between about 30° C and about 40 °C. In an embodiment step d) takes place between about 25 °C and about 35 °C. In an embodiment step d) takes place at about 25 °C , about 30 °C, about 35 °C, about 40 °C, or about 45 °C. In an embodiment step d) takes place at less than about 45 °C.

In an embodiment the compound of Formula I is produced in between about 70 % and 95% yield from step d). In an embodiment the compound of Formula I is produced in between about 70% and 90%, in between about 70% and 80%, or in between about 70% and 75% yield. In another embodiment the compound of Formula I is produce in between about 80% and about 95%, in between about 85% and about 95%, or in between about 90% and 95% yield. In another embodiment the compound of Formula I is produced in about 70%, about 75%, about 80%, about 85%, about 90%, or about 95% yield. In a further embodiment the compound of Formula I is produced in about 70% yield. In a further embodiment the compound of Formula I is produced in about 80% yield. In an embodiment the compound of Formula I is produced in at least about 65% yield, in at least 70% yield, in at least 75% yield, in at least about 80% yield, or at least about 85% yield from step d). In another embodiment Formula I is produced in at least about 70% yield from step d).

In an embodiment step d) is a coupling of a compound of Formula VII and a retinoic acid with TBTU, in DCM:2-MeTHF with triethylamine. In an embodiment step d) is a coupling of a compound of Formula VII and a retinoic acid with activation by TBTU, in DCM:2-MeTHF mixture with triethylamine at about 30° C. In an embodiment step d) is a coupling of a compound of Formula VII and a retinoic acid with TBTU, in DCM:2-MeTHF with triethylamine. In an embodiment step d) is a coupling of a compound of Formula VII and all-trans retinoic acid with activation by TBTU, in DCM:2-MeTHF with triethylamine at 30° C.

### Overall Process

In an embodiment the compound of Formula I is produced in between about 40% and about 80% overall yield from the compound of Formula III. In an embodiment the compound of Formula I is produced in between about 40% and about 70% yield, about 40% and about 60% yield, about 40% and about 50% yield, or about 40% and about 45% yield from the compound of Formula III. In an embodiment the compound of Formula I is produced in between about 50% and about 80%, about 60% and about 80% yield, about 70% and about 80% yield, about 75% and about 80% yield, about 80% and about 80% yield, or about 85% and about 80% yield from the compound of Formula III. In an embodiment the compound of Formula I is produced in about 60% yield from the compound of Formula III.In an embodiment the compound of Formula I is produced in at least about 40% yield, at least about 50% yield, at least about 60% yield, or at least about 65% yield from the compound of Formula II. In an embodiment the compound of Formula I is produced in at least about 50% yield from the compound of Formula III. In an embodiment the compound of Formula I is produced in at least about 60% yield from the compound of Formula III.

### EXAMPLES

### Example 1. Synthesis of dibenzyl (1-amino-15-oxo-4,7,10-trioxa-14-azaicosane-16,20-diyl)(S)-dicarbamate

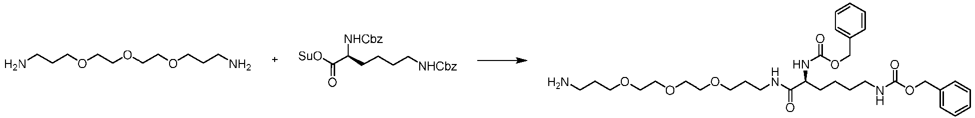

Charge 2-MeTHF (1.71 kg, 2.0 L, 2.0 L/kg) to Reactor 1 and begin agitation. Charge 2,5-dioxopyrrolidin-1-yl N²,N⁶-bis((benzyloxy)carbonyl)-*L*-lysinate (1.00 kg) to Reactor 1 at 20 °C. Charge DCM (7.96 kg, 6.0 L, 6.0 L/kg) at 20 °C. Begin agitation. Charge 2-MeTHF (4.27 kg, 5.0 L, 5.0 L/kg) to reactor 1. Charge 2-MeTHF (5.98 kg, 7.0 L, 7.0 L/kg) to Reactor 2 and begin agitation. Charge 3,3'-((oxybis(ethane-2,1-diyl))bis(oxy))bis(propan-1-amine) (3.45 kg, 8.0 eq., 3.45 kg/kg) to Reactor 2. Cool Reactor 2 to -25 to -12 °C (target -20 °C), and begin agitation. Charge the 2,5-dioxopyrrolidin-1-yl N²,N⁶-bis((benzyloxy)carbonyl)-*L*-lysinate solution from Reactor 1 to the diamine solution in Reactor 2 slowly at < -12 °C with good agitation and subsurface addition through dip tube. The addition of 2,5-dioxopyrrolidin-1-yl N²,N⁶-bis((benzyloxy)carbonyl)-*L*-lysinate to 3,3'-((oxybis(ethane-2,1-diyl))bis(oxy))bis(propan-1-amine) is exothermic. Maintain batch temperature ≤ -15 °C. Age the batch for no less than 5 minutes with continued agitation. Reduce agitation speed and warm Reactor 2 to 15-25 °C (target 20 °C). Charge 25 wt% brine (8.50 kg, 8.50 kg/kg), followed by water (4.80 kg, 4.80 kg/kg). The addition of brine is exothermic. Maintain batch temperature ≤ 25 °C. Agitate the batch for at least 30 minutes at 15-25 °C (target 20 °C). Split the phases, sending the bottom aqueous phase to waste. Charge 4 wt% citric acid (12.5 kg, 12.5 kg/kg) to Reactor 2. Agitate the batch for at least 30 minutes at 15 - 25 °C (target 20 °C). Split the phases, sending the product rich bottom aqueous phase to reactor 1. Charge the aqueous layer in Reactor 1 back to Reactor 2. Charge 2-MeTHF (11.53 kg, 13.5 L, 13.5 L/kg), followed by 10 M NaOH (~1,0 kg, ~0.75 L, 1.0 kg/kg) to pH 12-13 in Reactor 2. Split the phases, sending the bottom aqueous phase to waste. Charge 25 wt% brine (11.8 kg, 10.0 L, 11.8 kg/kg), followed by water (5.0 kg, 5.0 kg/kg) to Reactor 2. Split the phases, sending the bottom aqueous phase to waste. The batch was concentrated via distillation to a final volume of 4.5 L (4.5 L/kg) (~20 wt%), while maintaining temperature ≤ 40 °C. dibenzyl (1-amino-15-oxo-4,7,10-trioxa-14-azaicosane-16,20-diyl)(S)-dicarbamate was synthesized was produced in 88% yield.

### Example 2: Synthesis of tetrabenzyl ((5S,57S)-6,22,40,56-tetraoxo-11,14,17,25,28,31,34,37,45,48,51-undecaoxa-7,21,41,55-tetraazahenhexacontane-1,5,57,61-tetrayl)tetracarbamate

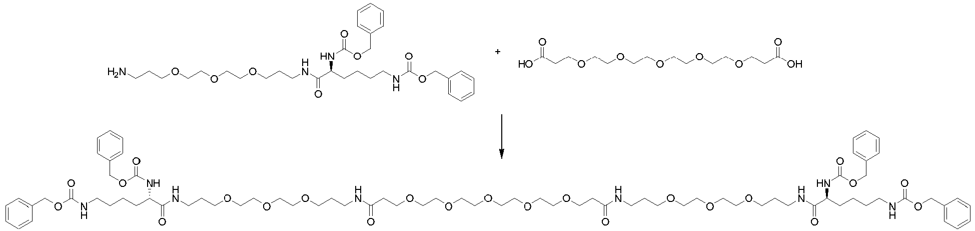

Charge dibenzyl (1-amino-15-oxo-4,7,10-trioxa-14-azaicosane-16,20-diyl)(*S*)-dicarbamate (2.05 eq.) as a process solution (e.g. 18.7 kg at 20 wt%) to Reactor 1 at 20 °C. Charge DCM (23.9 kg, 18.0 L, 18.0 L/kg) to Reactor 1 at 20 °C. Begin agitation and heat the batch to 15 - 25 °C (target 20 °C). Charge dicarboxylic acid 4,7,10,13,16-pentaoxanonadecanedioic acid (1.00 kg) to Reactor 1 at 20°C. Charge hydroxybenzotriazole hydrate (HOBT) (45.3 g, 0.100 eq.) to Reactor 1 at 20 °C. Charge EDAC (1.47 kg, 2.60 eq.). The addition of EDAC is exothermic. Maintain a batch temperature < 30 °C (target 20 °C). Age the batch at 15 - 25 °C (target 20 °C) for no less than 3 hours. Charge 2-MeTHF (43.0 kg, 50.0 L, 50.0 L/kg) at T_{J} = 20 °C.

Charge 5.00 wt% citric acid solution (31.0 kg, 30.0 L, 30.0 L/kg). Begin agitation and heat the batch to 30 - 40 °C (target 35 °C). Agitate the batch for 30 minutes at 30 - 40 °C (target 35 °C). Stop agitation and allow the phases to settle for at least 30 minutes. Split phases, sending the aqueous bottom phase to waste.

Charge 5.00 wt% potassium carbonate solution from Step 7 (20.9 kg, 20.0 L, 20.0 L/kg). Begin agitation and heat batch to 25 - 35 °C (target 30 °C). Agitate the batch for 30 minutes at 25 - 35 °C (target 30 °C). Stop agitation, and allow the phases to settle for at least 30 minutes. Split phases, sending the aqueous bottom phase to waste.

Charge 25 wt% sodium chloride brine (11.94 kg, 10.00 L, 10.00 L/kg). Charge water (11. 94 kg, 11. 94 L, 11. 94 L/kg). Agitate the batch for 30 minutes at 25 - 35 °C (target 30 °C). Stop agitation, and allow the phases to settle for at least 30 minutes. Split phases, sending the aqueous bottom phase to waste.

Begin agitation and cool batch to 0 °C. Concentrate the batch to a final volume of 12.5 L (12.5 L/kg) maintaining a batch temperature ≤ 50 °C. It is recommended that the distillation be performed at Tj ≤ 50 °C and P ≤ 80 mbar for stability of product and speed of distillation. Charge MeOH (9.9 kg, 12.5 L, 12.5 L/kg) via sprayball. Discharge through polish filter (≤ 10 µm pore size) at room temperature to store BMT-334119 process solution. tetrabenzyl ((5*S*,57*S*)-6,22,40,56-tetraoxo-11,14,17,25,28,31,34,37,45,48,51-undecaoxa-7,21,41,55-tetraazahenhexacontane-1,5,57,61-tetrayl)tetracarbamate is produced in 90-95% yield.

### Example 3: Synthesis ofN¹,N¹⁹-bis((S)-16,20-diamino-15-oxo-4,7,10-trioxa-14-azaicosyl)-4,7,10,13,16-pentaoxanonadecanediamide.

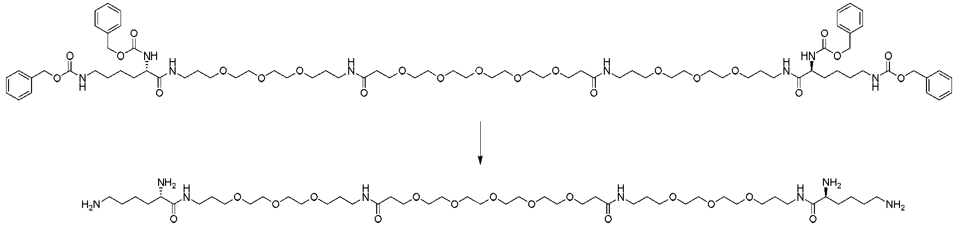

Set up 2 reactors (R1 & R2) with transfer line between them. 20 °C for both reactors. To R2, charge tetrabenzyl ((5*S*,57*S*)-6,22,40,56-tetraoxo-11,14,17,25,28,31,34,37,45,48,51-undecaoxa-7,21,41,55-tetraazahenhexacontane-1,5,57,61-tetrayl)tetracarbamate (1.00 kg) as a process solution in 1:1 2-MeTHF: MeOH. To R2, charge 1-methyl-1,4-cyclohexadiene (920 g, 1.10 L, 15.0 eq.). To R1, charge MeOH (4.11 kg, 5.2 L, 5.2 L/kg) and 2-MeTHF (0.512 kg, 0.6 L, 0.6 L/kg). Sparge R1 and R2 with N₂ for ≥ 20 min. Subsurface nitrogen flow achieved with dip tube feed close to impeller. Charge 5% Pd/C (100 g, 0.100 kg/kg) to R1 under inert conditions. Sparge R1 and R2 with N₂ for ≥ 20 min.

Heat batch in R1 to 40-50 °C (target 45 °C). Transfer R2 contents to R1 above surface. Stir for > 3.5 hr. At the end of the reaction age, sample for reaction completion. Filter sample immediately (≤ 1 *µm* pore size) to quench the reaction. *N*¹,*N*¹⁹ -bis((*S*)-16,20-diamino-15-oxo-4,7,10-trioxa-14-azaicosyl)-4,7,10,13,16-pentaoxanonadecanediamide is produced in 98% yield.

### Example 4: Synthesis of N¹,N¹⁹-bis((S,23E,25E,27E,29E)-16-((2E,4E,6E,8E)-3,7-dimethyl-9-(2,6,6-trimethylcyclohex-1-en-1-yl)nona-2,4,6,8-tetraenamido)-24,28-dimethyl-15,22-dioxo-30-(2,6,6-trimethylcyclohex-1-en-1-yl)-4,7,10-trioxa-14,21-diazatriaconta-23,25,27,29-tetraen-1-yl)-4,7,10,13,16-pentaoxanonadecanediamide

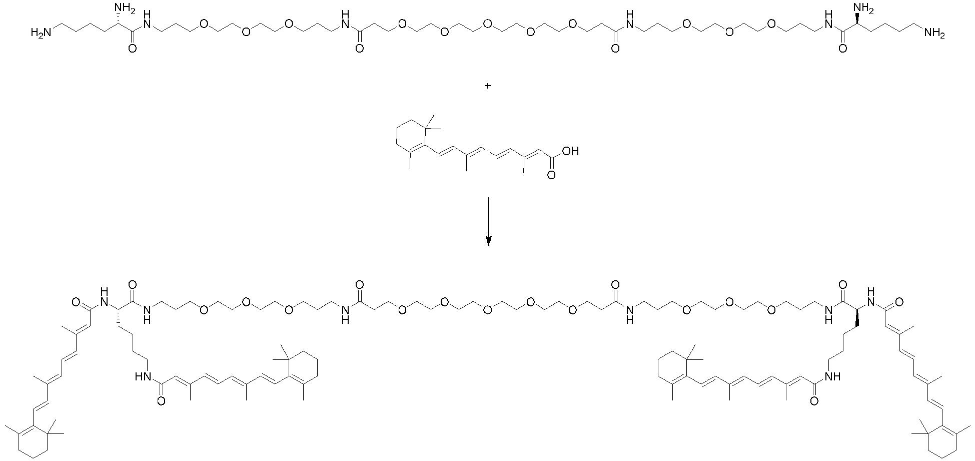

Charge water (10 kg, 10 L, 10 L/kg) to Reactor 1. Charge sodium bicarbonate (0.6 kg, 0.6 kg/kg) to Reactor 1. Agitate Reactor 1 until all solids are visibly dissolved. Charge *N*¹*,N*¹⁹-bis((*S*)-16,20-diamino-15-oxo-4,7,10-trioxa-14-azaicosyl)-4,7,10,13,16-pentaoxanonadecanediamide process solution in 1:1 2-MeTHF:MeOH (1.00 kg) to Reactor 2. Begin agitation and cool the batch to between 5-10 °C. Concentrate the *N*¹,*N*¹⁹-bis((*S*)-16,20-diamino-15-oxo-4,7,10-trioxa-14-azaicosyl)-4,7,10,13,16-pentaoxanonadecanediamide solution to a final volume of 5 L/kg (5 L) and solvent swap into 2-MeTHF, while maintaining batch temperature ≤ 45 °C. Heat the batch in Reactor 2 to 25-35 °C (target 30 °C). Charge DCM (15 L, 15 L/kg) to Reactor 2. Charge all-trans retinoic acid (1.50 kg, 5.00 eq.). Charge triethylamine (1.52 kg, 2.09 L, 15.0 eq.). Age the batch at 25-35 °C (target 30 °C) for at least 30 minutes until all solids are visibly dissolved. Charge TBTU (1.77 kg, 5.5 eq.). Age the batch at 25-35 °C (target 30° C) for no less than 4 hours.

Charge BHT (0.0018 kg, 0.0018 kg/kg). Charge 6 wt% sodium bicarbonate to Reactor 2 (10.6 kg, 10 L, 10 L/kg). Agitate the batch for at least 1 hour at 25-35 °C (target 30 °C). Stop agitation and allow the phases to settle for at least 30 minutes. Split the phases in Reactor 2, sending the bottom organic phase to Reactor 3 and the upper aqueous phase to waste. Begin agitation in Reactor 3 and cool the batch to between 5-10 °C. Concentrate the batch to 10 L/kg (10 L) and solvent swap into MeOH, while maintaining batch temperature ≤ 45 °C. Sample for IPC-4. Charge ethanol 200 proof (0.79 kg, 1 L, 1 L/kg). Purify product via C¹⁸ preparative chromatography using an ethanol:methanol gradient. *N*¹,*N*¹⁹-bis((*S*,23*E*,25*E*,27*E*,29*E*)-16-((2*E*,4*E*,6*E*,8*E*)-3,7-dimethyl-9-(2,6,6-trimethylcyclohex-1-en-1-yl)nona-2,4,6,8-tetraenamido)-24,28-dimethyl-15,22-dioxo-30-(2,6,6-trimethylcyclohex-1-en-1-yl)-4,7,10-trioxa-14,21-diazatriaconta-23,25,27,29-tetraen-1-yl)-4,7,10,13,16-pentaoxanonadecanediamide is produced in 85% yield.

## Claims

1. A process for synthesizing a compound of Formula I 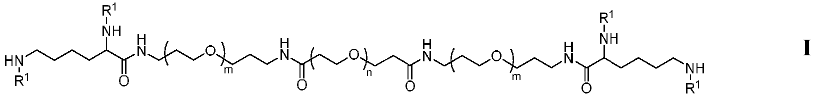 wherein, R¹ is a retinoid radical;
m is an integer from 1-6; and
n is an integer from 1-10
the process comprising,
a) reacting a compound of Formula II 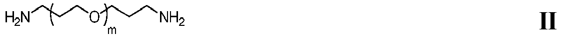 wherein, m is an integer from 1-6;
with a compound of Formula III 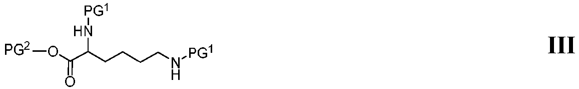
wherein, PG¹ and PG² are each independently a protecting group;
to form a compound of Formula IV 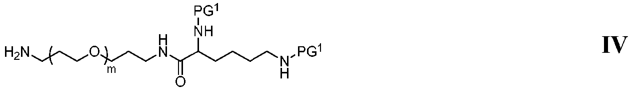
wherein m is an integer from 1-6; and
PG¹ is a protecting group;
b) reacting a compound of Formula IV with a compound of Formula V 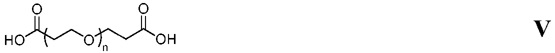 wherein, n is an integer from 1-10;
to form a compound of Formula VI 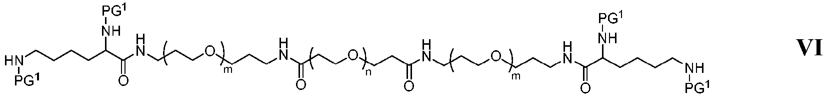
wherein, m is an integer from 1-6;
n is an integer from 1-10; and
PG¹ is a protecting group;
c) reacting a compound of Formula VI under hydrogenation conditions to form a compound of Formula VII 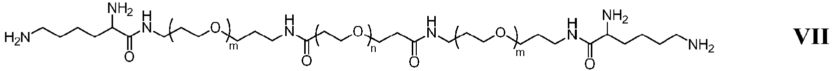 wherein m is an integer from 1-6; and
n is an integer from 1-10; and
d) reacting the compound of Formula VII under coupling conditions with a retinoid to form a compound of Formula I.

2. The process of claim 1, wherein the retinoid in step d) is selected from vitamin A, retinoic acid, tretinoin, adapalene, 4-hydroxy(phenyl)retinamide, retinyl palmitate, retinal, saturated retinoic acid, and saturated demethylated retinoic acid.

3. The process of claim 2, wherein the retinoid in step d) is retinoic acid.

4. The process of claim 1, wherein the hydrogenation conditions comprise reacting a compound of Formula I with H₂ and Pd/C.

5. The process of claim 1, wherein m is 3; or wherein n is 5.

6. The process of claim 1, wherein each PG¹ and PG² are independently selected from the group consisting of carboxybenzyl, *p*-methoxybenzyl carbonyl, *t*-butyloxycarbonyl, 9-fluorenylmethyloxycarbonyl, acetyl, trifluoroacetyl, benzoyl, benzyl, carbamate, *p-*methoxybenzyl, 3,4-dimethoxybenzyl, *p*-methoxybenzyl, tosyl, trichloroethyl chloroformate, (4-nitrophenyl)sulfonyl, methyl, ethyl, propyl, *n*-butyl, *t*-butyl, succinimide, 2,6-dimethylphenol, 2,6-diisopropylphenol, 2,6-di-*tert*-butylphenol, trimethylsilyl, allyl, 1,1-dimethylallyl, 2,2,2-trifluoroethyl, phenyl, and 4-methoxybenzyl.

7. The process of claim 6 wherein PG¹ is carboxybenzyl.

8. The process of claim 6 wherein PG² is succinimide.

9. The process of claim 1, wherein the compound of Formula II is

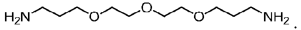

10. The process of claim 9, wherein the compound of Formula III is

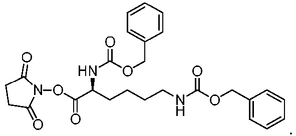

11. The process of claim 1 wherein a) is done at between -20 °C and 0 °C; or
wherein, the yield of a) is at least about 70%; or
wherein in a) the compound of Formula II is present in between about 2 and about 8 equivalents and the compound of Formula III is present in about 1 equivalent.

12. The process according to claim 1 wherein, a) further comprises the addition of citric acid after formation of the compound of Formula IV.

13. The process of claim 1, wherein the compound of Formula I is

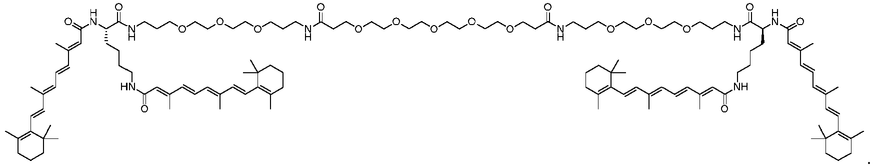

14. The process of claim 1, wherein the compound of Formula I is produced in at least about 50% yield from the compound of Formula III; or
wherein the compound of Formula IV is produced in a ratio of greater than 9:1 with a compound of Formula VIII

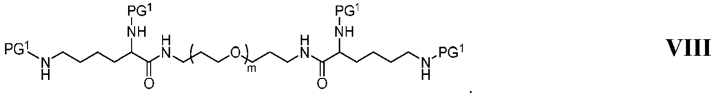

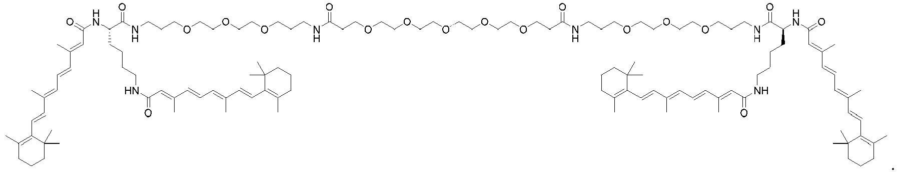

## Patentansprüche

1. Prozess zum Synthetisieren einer Verbindung mit der Formel I 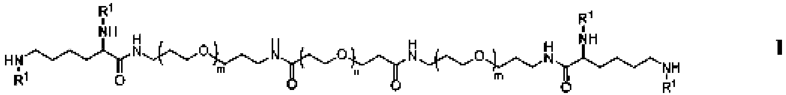 wobei R¹ ein Retinoidrest ist;
m eine Ganzzahl von 1-6 ist; und
n eine Ganzzahl von 1-10 ist
wobei der Prozess Folgendes umfasst:
a) Umsetzen einer Verbindung mit der Formel II 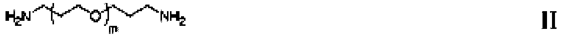 wobei m 1-6 ist;
mit einer Verbindung mit der Formel III 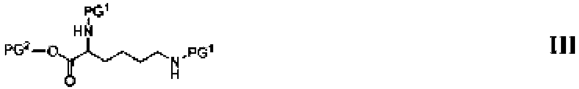
wobei PG¹ und PG² jeweils unabhängig eine Schutzgruppe sind;
zum Bilden einer Verbindung mit der Formel IV 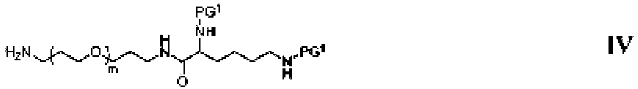
wobei m eine Ganzzahl von 1-6 ist; und
PG¹ eine Schutzgruppe ist;
b) Umsetzen einer Verbindung mit der Formel IV mit einer Verbindung mit der Formel V 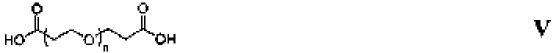 wobei n eine Ganzzahl von 1-10 ist;
zum Bilden einer Verbindung mit der Formel VI 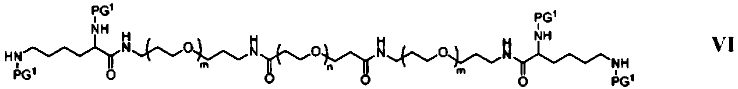
wobei m 1-6 ist;
n eine Ganzzahl von 1-10 ist.
PG¹ eine Schutzgruppe ist;
c) Umsetzen einer Verbindung mit der Formel VI unter Hydrierungsbedingungen zum Bilden einer Verbindung mit der Formel VII 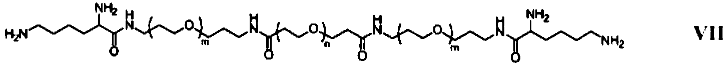 wobei m eine Ganzzahl von 1-6 ist; und
n eine Ganzzahl von 1-10 ist.
c) Umsetzen einer Verbindung mit der Formel VII unter Kopplungsbedingungen mit einem Retinoid zum Bilden einer Verbindung mit der Formel I.

2. Prozess nach Anspruch 1, wobei das Retinoid im Schritt d) ausgewählt ist aus Vitamin A, Retinsäure, Tretinoin, Adapalen, 4-Hydroxy(phenyl)retinamid, Retinylpalmitat, Retinal, gesättigter Retinsäure und gesättigter demethylierter Retinsäure.

3. Prozess nach Anspruch 2, wobei das Retinoid im Schritt d) Retinsäure ist.

4. Prozess nach Anspruch 1, wobei die Hydrierungsbedingungen das Umsetzen einer Verbindung mit der Formel I mit H₂ und Pd/C umfassen.

5. Prozess nach Anspruch 1, wobei m 3 ist; oder wobei n 5 ist.

6. Prozess nach Anspruch 1, wobei jedes PG¹ und jedes PG² unabhängig ausgewählt sind aus der Gruppe, bestehend aus Carboxybenzyl, p-Methoxybenzylcarbonyl, *t-*Butyloxycarbonyl, 9-Fluorenylmethoxycarbonyl, Acetyl, Trifluoracetyl, Benzoyl, Benzyl, Carbamat, p-Methoxybenzyl, 3,4-Dimethoxybenzyl, p-Methoxybenzyl, Tosyl, Triclorethylchlorformiat, (4-Nitrophenyl)sulfonyl, Methyl, Ethyl, Propyl, *n*-Butyl, *t*-Butyl, Succinimid, 2,6-Dimethylphenol, 2,6-Diisopropylphenol, 2,6-Di-*tert*-butylphenol, Trimethylsilyl, Allyl, 1,1-Dimethylallyl, 2,2,2-Trifluorethyl, Phenyl und 4-Methoxybenzyl.

7. Prozess nach Anspruch 6 wobei PG¹ Carboxybenzyl ist.

8. Prozess nach Anspruch 6 wobei PG² Succinimid ist.

9. Prozess nach Anspruch 1, wobei die Verbindung mit der Formel II Folgendes ist:

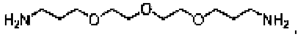

10. Prozess nach Anspruch 9, wobei die Verbindung mit der Formel III Folgendes ist:

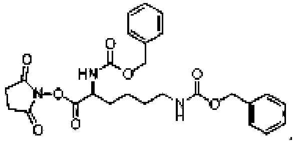

11. Prozess nach Anspruch 1, wobei a) bei einer Temperatur zwischen -20 °C und 0 °C durchgeführt wird; oder
wobei die Ausbeute von a) mindestens ungefähr 70 % beträgt; oder
wobei in a) die Verbindung mit der Formel II in zwischen ungefähr 2 und ungefähr 8 Äquivalenten vorhanden ist und die Verbindung mit der Formel III in ungefähr 1 Äquivalent vorhanden ist.

12. Prozess nach Anspruch 1, wobei a) ferner die Zugabe von Zitronensäure nach Bildung der Verbindung mit der Formel IV umfasst.

13. Prozess nach Anspruch 1, wobei die Verbindung mit der Formel I Folgendes ist:

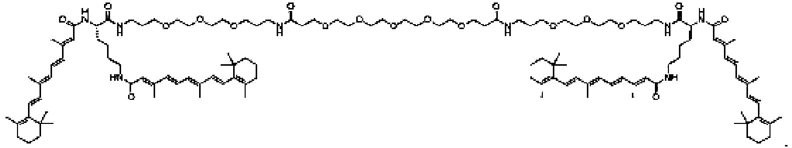

14. Prozess nach Anspruch 1, wobei die Verbindung mit der Formel I mit einer Ausbeute von mindestens ungefähr 50 % aus der Verbindung mit der Formel III hergestellt wird; oder
wobei die Verbindung mit der Formel IV in einem Verhältnis von größer als 9:1 mit einer Verbindung mit der Formel VIII hergestellt wird

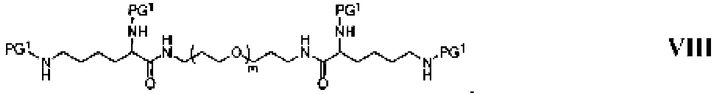

## Revendications

1. Processus pour la synthèse d'un composé de Formule I 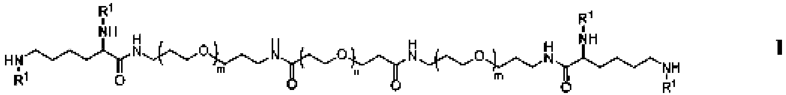 dans lequel R¹ est un radical rétinoïde ;
m est un nombre entier de 1 à 6 ; et
n est un nombre entier de 1 à 10
le processus comprenant,
a) la mise en réaction d'un composé de Formule II 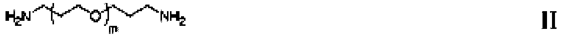 dans lequel m est 1 à 6 ;
avec un composé de Formule III 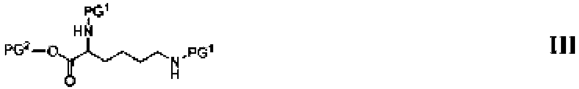
dans lequel PG¹ et PG² sont chacun indépendamment un groupe protecteur;
pour former un composé de Formule IV 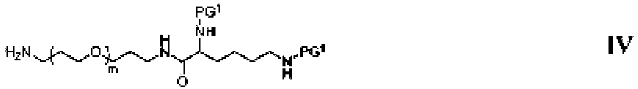
dans lequel m est un nombre entier de 1 à 6 ; et
PG¹ est un groupe protecteur ;
b) la mise en réaction d'un composé de Formule IV avec un composé de Formule V 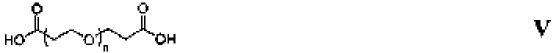 dans lequel n est un nombre entier de 1 à 10 ;
pour former un composé de Formule VI 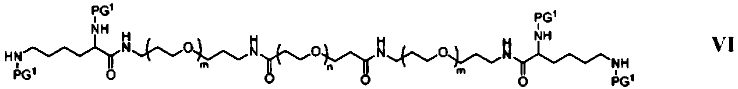
dans lequel m est 1 à 6 ;
n est un nombre entier de 1 à 10 ; et
PG¹ est un groupe protecteur ;
c) la mise en réaction d'un composé de Formule VI dans des conditions d'hydrogénation pour former un composé de Formule VII 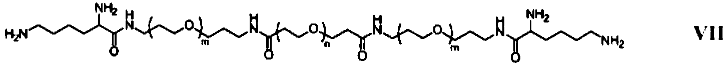 dans lequel m est un nombre entier de 1 à 6 ; et
n est un nombre entier de 1 à 10 ; et
d) la mise en réaction du composé de Formule VII dans des conditions de couplage avec un rétinoïde pour former un composé de Formule I.

2. Processus selon la revendication 1, dans lequel le rétinoïde est choisi parmi : vitamine A, acide rétinoïque, trétinoïne, adapalène, 4-hydroxy(phényl)rétinamide, palmitate de rétinyle, rétinal, acide rétinoïque saturé et acide rétinoïque déméthylé saturé.

3. Processus selon la revendication 2, dans lequel le retinoïde en étape d) est un acide rétinoïque.

4. Processus selon la revendication 1, dans lequel les conditions d'hydrogénation comprennent la mise en réaction d'un composé de Formule I avec H₂ et Pd/C.

5. Processus selon la revendication 1, dans lequel m est 3 ; ou dans lequel n est 5.

6. Processus selon la revendication 1, dans lequel PG¹ et PG² sont chacun indépendamment choisis parmi le groupe consistant en : carboxybenzyle, p-méthoxybenzylcarbonyle, *t*-butyloxycarbonyle, 9-fluorénylmétholoxycarbonyle, acétyle, trifluoroacétyle, benzoyle, benzyle, carbamate, *p*-méthoxybenzyle, 3,4-diméthoxybenzyle, *p*-méthoxybenzyle, tosyle, chloroformate de trichloroéthyle, (4-nitrophényl)sulfonyle, méthyle, éthyle, propyle, *n*-butyle, *t*-butyle, succinimide, 2,6-diméthylphénol, 2,6-diisopropylphénol, 2,6-di-*tert*-butylphénol, triméthylsilyle, allyle, 1,1-diméthylallyle, 2,2,2-trifluoroéthyle, phényle et 4-méthoxybenzyle.

7. Processus selon la revendication 6, dans lequel PG¹ est un carboxybenzyle.

8. Processus selon la revendication 6, dans lequel PG²est un succinimide.

9. Processus selon la revendication 1, dans lequel le composé de Formule II est

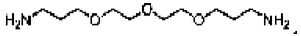

10. Processus selon la revendication 9, dans lequel le composé de Formule III est

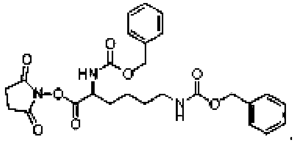

11. Processus selon la revendication 1, dans lequel a) est effectué à une température entre -20 °C et 0 °C ; ou
dans lequel, le rendement de a) est d'au moins environ 70 % ; ou
dans lequel, dans a), le composé de Formule Il est présent à raison d'entre environ 2 et environ 8 équivalents et le composé de Formule III est présent à raison d'environ 1 équivalent.

12. Processus selon la revendication 1, dans lequel a) comprend en outre l'ajout d'acide citrique après la formation du composé de Formule IV.

13. Processus selon la revendication 1, dans lequel le composé de Formule I est

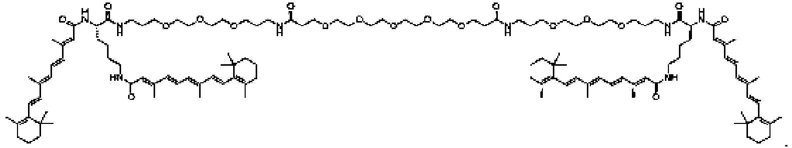

14. Processus selon la revendication 1, dans lequel le composé de Formule I est produit dans un rendement d'au moins environ 50 % à partir du composé de Formule III ; ou
dans lequel le composé de Formule IV est produit dans un rapport de plus de 9:1 avec un composé de Formule VIII
